# FASCICULE DE BREVET EUROPEEN

(11) **EP 1 121 414 B1**
(45) Date de publication et mention de la délivrance du brevet: **02.04.2003**
(21) Numéro de dépôt: 98949075.0
(22) Date de dépôt: 19.10.1998
(51) Int. Cl.: C12M 1/00, C12M 1/36

(54) **PROCEDE POUR AMELIORER LE RENDEMENT D'UN PHOTOBIOREACTEUR**
VERFAHREN ZUR VERBESSERUNG DER AUSGEBEUTE EINES PHOTOBIOREAKTORS
METHOD FOR IMPROVING THE PERFORMANCE OF A PHOTOBIOREACTOR

(43) Date de publication de la demande: 08.08.2001
(73) Titulaire: INSTITUT FRANCAIS DE RECHERCHE POUR L'EXPLOITATION DE LA MER (IFREMER), 92138 Issy-les-Moulineaux Cedex (FR)
(72) Inventeur: MULLER-FEUGA, Arnaud, F-44000 Nantes (FR)
(74) Mandataire: Dronne, Guy
(86) Numéro de dépôt international: FR9802234
(87) Numéro de publication internationale: WO00023562

(56) Documents cités:
- DE-A- 2 658 894
- FR-A- 2 342 785
- FR-A- 2 576 034
- US-A- 3 504 185
- US-A- 3 855 120
- US-A- 5 589 935
- DATABASE WPI Section Ch, Week 9343 Derwent Publications Ltd., London, GB; Class D16, AN 93-338908 XP002053121 & JP 05 244930 A (EBARA INFILCO KK), 24 septembre 1993 (1993-09-24)
- PATENT ABSTRACTS OF JAPAN vol. 015, no. 496 (C-0894), 16 décembre 1991 (1991-12-16) & JP 03 216180 A (TOSHIBA CORP), 24 septembre 1991 (1991-09-24)

## Description

L'invention se rapporte au domaine des photobioréacteurs, c'est-à-dire des appareils qui permettent la croissance contrôlée de micro-organismes photosynthétiques.

Elle concerne plus précisément un procédé pour améliorer la vitesse de croissance de microorganismes phototrophes d'une culture en suspension dans un milieu réactionnel liquide, procédé selon lequel on soumet la culture à une réaction photosynthétique activée par des rayonnements traversant une paroi transparente d'une chambre d'épaisseur d d'un photobioréacteur dans lequel circule le milieu réactionnel.

Il est connu que les performances des systèmes de production de microorganismes phototrophes varient avec les espèces cultivées, et pour une espèce donnée, avec la concentration et l'épaisseur de la couche de culture soumise aux rayonnements. Les variations interspécifiques sont dues au fait que certaines espèces sont plus pénalisées que d'autres par un excès de lumière, ont une meilleure capacité à utiliser les faibles intensités lumineuses, peuvent être cultivées en de plus fortes concentrations que d'autres.

Les variations de performances intraspécifiques sont essentiellement liées à l'épaisseur de la couche de culture soumise aux rayonnements dans la chambre et à la concentration de la culture. On a pu noter que l'absorbance ou densité optique de la culture de micro-organismes est plus ou moins conservée quelles que soient l'épaisseur d de la couche et la concentration c de la culture. Ceci revient à dire que d x c est égal à une constante exprimée en grammes de poids sec de microorganismes par mètre carré de surface photosynthétique, et que l'on appelle aussi densité surfacique. La valeur de cette constante varie entre 10 et 40 g/m² selon les espèces.

On voit donc que l'épaisseur d moyenne des chambres ou canalisations dans lesquelles s'effectue la réaction photonique devrait changer avec l'espèce de microorganismes cultivée pour une concentration souhaitée de la culture.

Pour une espèce donnée, et une concentration élevée, il faudrait une épaisseur d faible, afin de rendre la lumière accessible à la population de microorganismes en suspension dans le milieu réactionnel liquide et circulant dans la chambre du réacteur, afin d'améliorer le rendement de la réaction. Mais une épaisseur d faible peut entraîner des pertes de charges conséquentes dans le circuit.

Il est toujours bon d'accroître la concentration finale des microorganismes dans le milieu réactionnel liquide, afin de limiter les volumes de liquide à traiter en amont (préparation de milieu nutritif) et à l'aval (concentration de la biomasse, traitement du milieu clarifié avant rejet). Pour améliorer la performance des photobioréacteurs, il faudrait donc pouvoir modifier l'épaisseur d des chambres du réacteur, en fonction de l'espèce cultivée. Or, ceci n'est pas aisément réalisable dans la pratique.

US-A-3 504 185, qui est considéré comme l'état de la technique le plus proche de l'invention, décrit un photobioréacteur et un procédé pour mesurer et contrôler la croissance de microorganismes phototropes dans un milieu de réaction. Selon le procédé, la densité de la population de microorganismes dans la réaction et par conséquent la vitesse de croissance des microorganismes est ajustée par l'ajout d'une culture fraîche dans le milieu de réaction et contrôlée au moyen de mesures du voltage électrique dans le milieu.

JP-A-03 216180 décrit un bioréacteur par assimilation et retrait de CO₂ par photosynthèse. Un tube de verre rempli de billes de verre est disposé coaxialement dans le bioréacteur afin de disperser la lumière entrant dans ce tube vers le milieu réactionnel.

FR-A-2 576 034 décrit un procédé et une bioréaction de production de matières premières hydrocarbonées par photosynthèse. On ajoute des billes dans le milieu réactionnel, lesdites billes ayant une densité inférieure ou supérieure à celui du milieu réactionnel, afin d'assurer l'agitation du milieu aqueux et en même temps le nettoyage des parois du photobioréacteur.

FR-A-2 342 785 décrit un procédé pour améliorer le transport des matières dans un milieu liquide, dans lequel on ajoute, dans le milieu liquide, des billes ayant une densité supérieure à celle du milieu réactionnel.

Le but de l'invention est de proposer un procédé facile à mettre en oeuvre qui permette d'adapter un photobioréacteur donné du type à recirculation et fonctionnement continu, en fonction de l'espèce de microorganismes cultivés et de la concentration souhaitée de la culture, quelle que soit l'épaisseur d des chambres du réacteur, afin d'améliorer la vitesse de croissance des microorganismes.

L'invention atteint son but par le fait que dans le procédé proposé on règle l'absorbance de la culture en ajoutant au milieu réactionnel des particules transparentes ou réfléchissantes ayant une densité sensiblement égale à celle du milieu réactionnel, le pourcentage volumique desdites particules dans le milieu réactionnel étant fonction de l'espèce de microorganismes cultivés, de l'épaisseur d de la chambre et de la concentration finale souhaitée de la culture dans le milieu réactionnel.

Les particules accompagnent le milieu réactionnel de façon homogène dans sa circulation. Elles ont pour effet de diminuer la longueur du chemin optique dans les chambres. Augmenter le pourcentage volumique de particules dans le milieu réactionnel revient à diminuer l'épaisseur d de la couche dans la chambre sans augmenter les pertes de charges, ou à diluer la culture sans rajouter d'eau, ce qui n'irait pas dans le sens souhaité du point de vue de la récolte de biomasse.

Un autre effet attendu des particules est le nettoyage des parois optiques du réacteur quelle que soit sa forme.

La quantité de particules est notamment fonction de la concentration finale souhaitée de la biomasse à obtenir.

Dans un fonctionnement en continu d'un bioréacteur, cette concentration reste constante. Toutefois, au cours du lancement d'une nouvelle culture et de la montée en charge du système, la concentration est nettement inférieure à la concentration finale souhaitée. Les micro-organismes sont alors soumis à une trop forte intensité de rayonnement, ce qui peut être préjudiciable au rendement.

Selon une autre caractéristique de procédé selon l'invention, on mesure la turbidité du milieu réactionnel et on règle l'intensité des rayonnements en fonction de la mesure de la turbidité.

Avantageusement, les particules ont des formes massives pour éviter les effets de portance et de superposition. Le diamètre des cercles exinscrits des particules est compris, de préférence, entre 0,5mm et 10mm. On utilise, par exemple, des particules, réalisées en enrobant des microbilles de verre creuses dans une matière plastique transparente dans des proportions telles que la densité du composite obtenu soit égale à celle du milieu réactionnel.

L'invention concerne également un photobioréacteur du type à recirculation et à fonctionnement continu pour la mise en oeuvre du procédé selon l'invention.

Ce photobioréacteur comporte une boucle fermée dans laquelle peut circuler une culture en suspension dans un milieu réactionnel liquide, au moins un chambre d'épaisseur d prévue dans ladite boucle et délimitée par une paroi transparente, des moyens pour émettre des rayonnements à travers ladite paroi transparente, des moyens pour introduire des particules dans ladite boucle et des moyens pour assurer la rétention desdites particules dans ladite boucle.

D'autres avantages et caractéristiques de l'invention ressortiront à la lecture de la description suivante faite à titre d'exemple et en référence aux dessins annexés dans lesquels :
la figure 1 montre la courbe de croissance des microorganismes phototrophes d'une espèce donnée en fonction de l'énergie irradiante des rayonnements reçus par le milieu réactionnel ;
la figure 2 montre schématiquement la mise en oeuvre du procédé selon l'invention dans un photobioréacteur ;
la figure 3 montre en perspective un photobioréacteur pour la mise en oeuvre de l'invention ;
la figure 4 montre un ensemble de chambres lumineuses du photobioréacteur de la figure 3 ; et
la figure 5 est une coupe axiale verticale d'une chambre lumineuse du photobioréacteur de la figure 3.

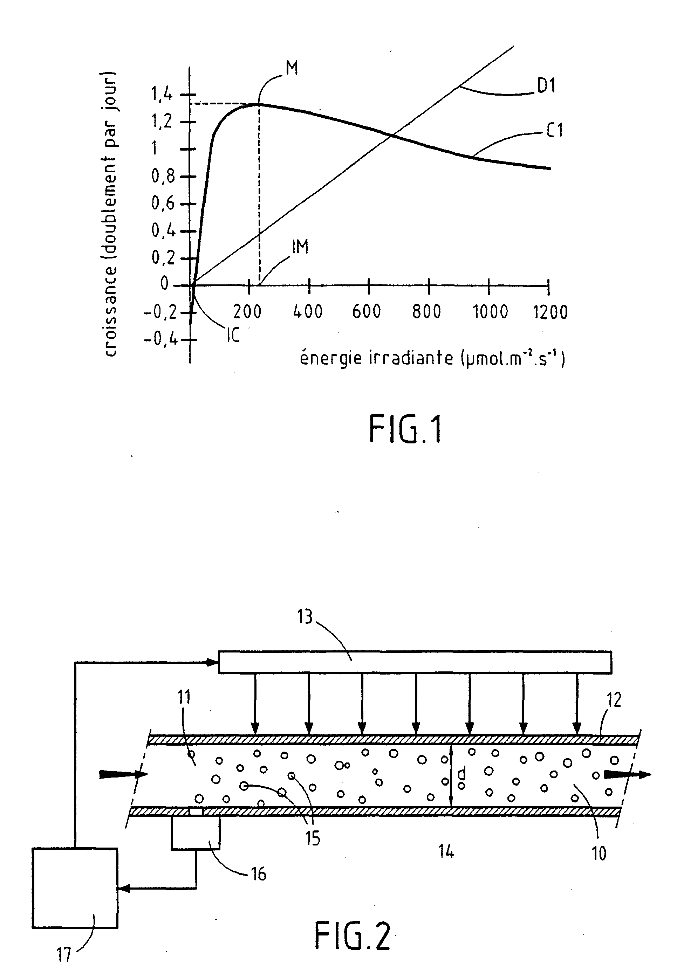

Les organismes phototrophes ont pour particularité d'être sensibles à l'excès de lumière comme à son insuffisance. En d'autres termes, alors que dans les processus physio-chimiques on ne vise qu'à accroître les photons introduits dans le milieu réactionnels, dans la culture des microorganismes phototrophes, il faut veiller à ce que les photons soient fournis à la réaction dans une fourchette d'énergie étroite. Pour illustrer ce propos, on se reportera à la figure 1 ou l'on voit que l'efficacité de la réaction photosynthétique, représentée par la courbe C1, présente un maximum M alors que l'efficacité d'une réaction photochimique, représentée par la droit D1, est approximativement proportionnelle à l'énergie disponible. L'ordonnée Gm du point M correspond à la vitesse de croissance maximale de la culture. Cette vitesse est obtenue pour une intensité irradiante optimale Im. On constate que lorsque l'intensité irradiante est inférieure à Ic, Ia population de microorganismes diminue. Les constantes Im, Ic, Gm sont obtenues expérimentalement et caractérisent chaque espèce d'algue. Lorsque l'intensité irradiante est supérieure ou inférieure à Im, la vitesse de croissance est inférieure à Gm. On voit donc l'intérêt qu'il y a à soumettre la population de microorganismes circulant dans un photobioréacteur à l'intensité irradiante optimale Im caractéristique de chaque espèce d'algues.

L'invention se donne pour but d'adapter un photobioréacteur du type à recirculation et à fonctionnement continu en fonction des caractéristiques des différents organismes cultivés.

La figure 2 montre schématiquement un réacteur de photosynthèse qui comporte une chambre 10 dans laquelle circule un milieu réactionnel liquide 11. La chambre est délimitée, sur l'une de ses faces, par une paroi transparente 12 qui reçoit des rayonnements émis par une source lumineuse 13, sur l'autre face, par une paroi 14 étanche à la lumière. Les parois 12 et 14 sont séparées d'une distance d. Afin de rendre la lumière accessible à la population de microorganismes en suspension dans le milieu réactionnel 11, et de permettre une concentration élevée d'organismes dans le liquide réactionnel, des particules 15 sont introduits dans le milieu réactionnel 14. Ces particules 15 sont transparentes ou réfléchissantes et ont une densité sensiblement égale à celle du milieu réactionnel, afin de l'accompagner de façon homogène dans sa circulation.

Le pourcentage volumique de particules 15 dans le milieu réactionnel est fonction de l'espèce de microorganismes cultivés, de l'épaisseur d de la chambre 10, de la concentration c finale souhaitée de microorganismes dans le milieu réactionnel et de la puissance de rayonnement de la source lumineuse 13, afin que l'énergie irradiante soit la plus voisine possible de Im dans la chambre 10.

La référence 16 représente un dispositif de mesure de turbidité du milieu réactionnel 11 qui agit sur un dispositif de réglage 17 de la puissance rayonnante émise par la source lumineuse 13.

Lorsque la concentration en microorganismes dans le milieu réactionnel 11 est inférieure à la concentration finale souhaitée en phase de production en continu, la puissance rayonnante de la source lumineuse est adaptée par le dispositif de réglage 17, afin d'obtenir une vitesse de croissance maximale.

Cette situation se produit notamment lors du lancement d'une nouvelle culture ou de la montée en charge du réacteur.

Les particules 15 de forme massive peuvent être avantageusement réalisées en enrobant des billes de verre creuses dans une matière plastique dans des proportions telles que la densité du composite obtenu soit égale à celle du milieu réactionnel. La matière plastique est choisie en fonction des contraintes d'exploitation. Le polyester est particulièrement adapté, car il conserve ses qualités mécaniques dans les plages de température élevées. La seule restriction concernant les particules 15 est leur tenue à la température dans le cas où la stérilisation initiale du réacteur se fait à la vapeur.

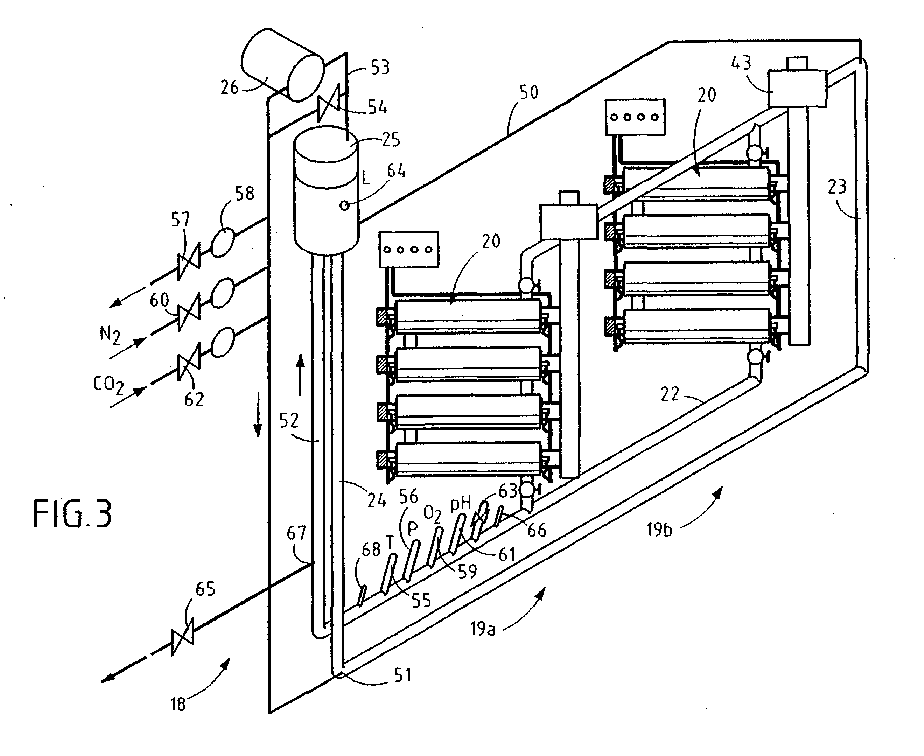

Les figures 3 à 5 montrent un photobioréacteur 18 du type à recirculation et fonctionnement continu, adapté pour mettre en oeuvre le procédé selon l'invention.

Ce photobioréacteur 18 comporte plusieurs ensembles 19a, 19b de chambres lumineuses 20, disposés en parallèle entre un distributeur 22 de milieu réactionnel liquide et un collecteur de sortie 23. Le collecteur de sortie 23 est raccordé au pied d'un élévateur à gaz 24 qui mène à un réservoir 25 dans lequel se fait la séparation gaz-liquide.

Le liquide retourne vers le distributeur 22, tandis que les gaz collectés passent dans un compresseur 26 et sont réinjectés en partie basse de la canalisation verticale de l'élévateur 24.

Chaque ensemble 19a, 19b comporte plusieurs chambres lumineuses 20, disposées les unes au-dessus des autres, et alimentées en série.

Chaque chambre lumineuse 20 comporte un cylindre 30, partiellement fermé à ses extrémités, et au centre duquel se trouve une source de lumière artificielle 31, de préférence un tube fluorescent qui a l'avantage d'un bon rendement de transformation de l'énergie électrique en lumière visible.

Le tube fluorescent 31 est disposé coaxialement dans un tube transparent 32. Des joints 34 assurent l'étanchéité entre le tube transparent 32 et le cylindre 30 aux deux extrémités de ce dernier, de telle sorte que la cavité ou chambre 10 ainsi réalisée puisse recevoir une culture de microorganismes 11 entrant par l'orifice 35 et sortant par l'orifice 36. Ces microorganismes reçoivent ainsi l'énergie photonique produite par le tube fluorescent 31 pendant tout leur parcours le long de la chambre 10.

L'épaisseur d de la culture au droit de la source lumineuse 31 est égale à la différence des rayons intérieur du cylindre 30 et extérieur de tube transparent 32. Cette épaisseur est choisie suffisamment grande, de telle sorte que l'énergie est complètement utilisée avant d'atteindre la paroi du cylindre 30. Elle est comprise entre quelques millimètres et quelques centimètres. Toutefois, on peut réaliser un chambre 10 dont la paroi externe est réfléchissante, par exemple en acier inoxydable, pour augmenter sensiblement la longueur du chemin optique avant extinction complète. On pourrait aussi sabler la surface réfléchissante pour rendre la lumière réfléchie isotrope et réduire les retours radiaux vers le tube transparent 32.

Pour augmenter les chances que l'ensemble des microorganismes reçoivent de la lumière, la culture circule dans la chambre 10 dans un régime turbulent.

La photosynthèse s'accompagnant d'une production d'oxygène, on pendra soin de faire en sorte que le milieu réactionnel 11 puisse s'échapper par l'orifice de sortie 36 en disposant celle-ci vers le haut. Les figures 3 à 5 montrent une disposition horizontale de la chambre 10. D'autres réalisations peuvent comporter des chambres verticales ou obliques.

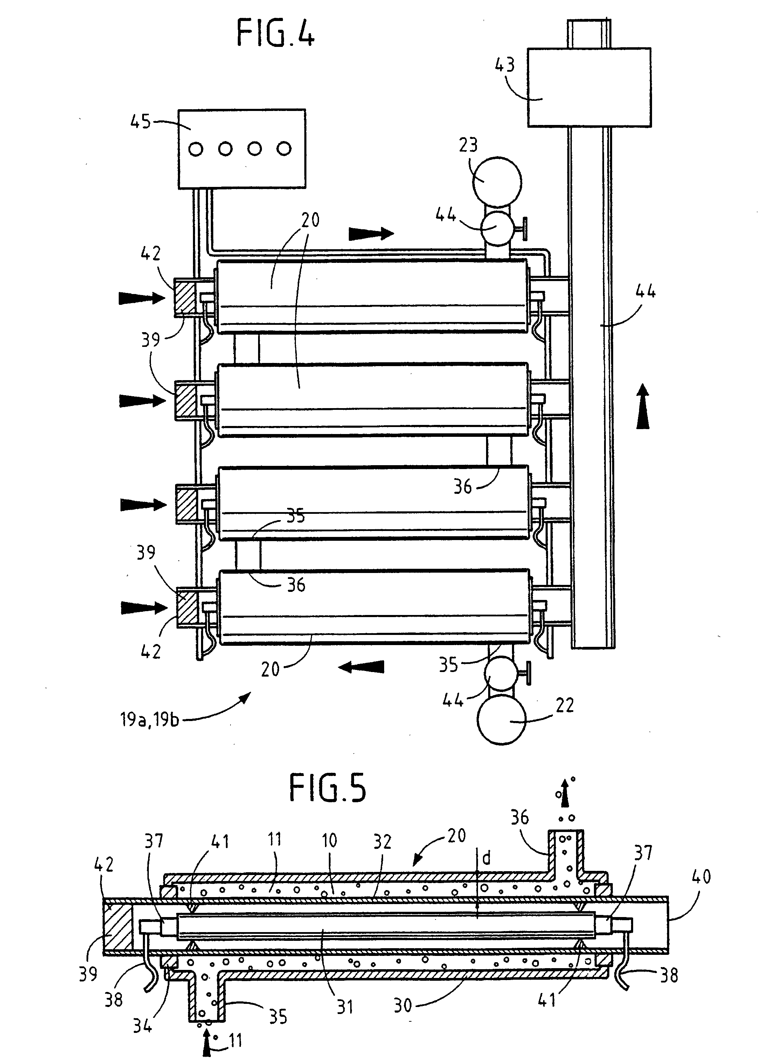

Les connecteurs 37 du tube fluorescent 31 sont raccordés à une alimentation électrique par l'intermédiaire de conducteurs 38 qui traversent le tube transparent 32 ou son prolongement, de telle sorte que les extrémités 39 et 40 soient libres. Le tube fluorescent 31 est centré dans le tube transparent 32 au moyen de cales 41. Les diamètres du tube fluorescent 31 et du tube transparent 32 sont tels qu'un passage d'air est réalisé pour assurer le refroidissement du tube fluorescent 31 et de la culture par échange conductif au travers du tube transparent 32. Pour éviter que des poussières ambiantes ne se déposent sur les surfaces optiques lors du passage de l'air, elles peuvent être utilement retenues au moyen du filtre 42 disposé à l'entrée du tube transparent 32. L'autre extrémité 41 est reliée à un extracteur 43, montré sur la figure 4, qui assure l'aspiration de l'air ambiant.

Comme les tubes fluorescents 31 dissipent une grande partie de l'énergie qu'ils consomment sous forme de chaleur, la culture subirait une augmentation de température, si une source froide n'est pas interposée sur la boucle. Les températures optimales de croissance des microorganismes photosynthétiques étant généralement supérieures aux températures que l'on trouve dans les locaux climatisés, le réacteur peut être avantageusement placé dans de tels locaux, dont le système de climatisation déjà en place assurerait la régulation de la source froide en même temps que le confort du personnel d'exploitation. La température de la culture est régulée par pilotage de l'extracteur 43.

La figure 4 montre comment plusieurs chambres lumineuses 20 peuvent être assemblées entre elles pour constituer un ensemble 19a, 19b, sans limiter pour autant les possibilités de mise en oeuvre à ces seules dispositions. Quatre chambres lumineuses 20 telles que décrites ci-dessus sont disposées les unes au-dessus des autres, et reliées entre elles en série par leurs extrémités, de telle sorte que la culture parcoure un chemin en zigzag ascendant depuis le distributeur 22 jusqu'au collecteur 23. L'oxygène formé au cours de la photosynthèse est ainsi entraînée dans la partie supérieure du collecteur 22 sans possibilité de s'accumuler sur ce parcours.

Ainsi que cela est montré sur la figure 3, le distributeur 22 et le collecteur 23 peuvent être branchés à plusieurs ensembles19a, 19b. Des vannes 44 permettent d'isoler des ensembles 19a, 19b et de les rendre ainsi indépendants les uns des autres. Ceci est utile en particulier lors du lancement d'une culture, pour mettre en service les ensembles 19a, 19b de façon progressive en fonction de la concentration et du volume des inoculum ou en cas de dysfonctionnement d'un assemblage en cours d'exploitation.

L'air ambiant entre dans les tubes transparents 32 par les extrémités 39 au travers des filtres 42. Puis, il est collecté par la canalisation 44 reliée à l'extracteur 43. Une alimentation électrique 45 distribue l'énergie aux quatre tubes fluorescents de chaque ensemble 19a, 19b.

Ainsi qu'on le voit sur la figure 3, les deux ensembles 19a, 19b sont alimentés en parallèle de bas en haut par le distributeur 22. Le collecteur 23 reprend la culture selon un circuit destiné à éviter l'alimentation préférentielle du premier ensemble 19a, et l'amène au pied de élévateur à gaz 24. La canalisation de faible diamètre 50 qui relie la zone supérieure du collecteur 23 au réservoir 25 est destiné à éviter l'accumulation d'oxygène en partie haute du collecteur 23, ce qui aurait pour effet de diminuer sa section et de ralentir la circulation du milieu réactionnel.

L'élévateur à gaz 24 a pour fonction d'assurer la circulation du milieu réactionnel. L'injection du gaz comprimé par le compresseur 26 au pied 51 de la colonne verticale 24 a pour effet d'entraîner le liquide contenu dans la colonne 24 vers le haut. La séparation gaz-liquide a lieu dans le réservoir 26 situé en partie haute de la colonne 24. La culture retourne vers le distributeur 22 par une canalisation verticale 52, tandis que les gaz sont évacués vers le haut ans une canalisation 53.

Les gaz sont recyclés, afin de parfaire les échanges gaz-liquides et de diminuer ainsi la consommation aux stricts besoins de la réaction. Pour cela, les gaz collectés passent dans le compresseur 26 et sont réinjectés en partie basse 51 de la colonne 24 de l'élévateur à gaz. Une dérivation permet de régler le débit injecté en agissant sur la vanne 54.

Nous allons maintenant décrire les différentes régulations du photobioréacteur 18 décrit ci-dessus. Par souci de clarté, les boîtiers de régulation ne sont pas figurés sur les dessins et les capteurs ne sont pas représentés en détail.

Le capteur de température 55, noté T, permet le pilotage du fonctionnement des extracteurs d'air 43 de chaque ensemble 19a, 19b.

L'injection de gaz au pied 51 de l'élévateur à gaz 24 a pour effet d'augmenter la pression dans le confinement. Une régulation de pression à une valeur supérieure de 0,1 bar à l'ambiant a pour objet d'éviter qu'en cas de fuite, les échanges entre l'extérieur et l'intérieur du confinement se produisent, ce qui risquerait de contaminer la culture. Un capteur de pression 56, noté P, commande l'ouverture de la vanne 57, ce qui a pour effet de laisser d'échapper les gaz contenus dans le réacteur. Un filtre 58 coupant à 0,22 micron permet d'éviter les contaminations à contre-courant.

L'accumulation d'oxygène dans le confinement est préjudiciable à la culture et doit être contrôlée. Pour cela, l'oxygène est déplacé par injection d'air ou d'azote dans le circuit gazeux, de telle sorte que la concentration en oxygène ne dépasse pas un seuil compris entre une et trois fois la saturation. Dans l'exemple de réalisation de la figure 3, le capteur 59, noté 02, commande l'ouverture de l'électrovanne 60 qui a pour effet de laisser entrer de l'azote dans le circuit gazeux du réacteur. L'azote passe au préalable par un filtre coupant à 0,22 microns qui permet d'éviter les contaminations du milieu réactionnel.

Le dioxyde de carbone est la source la plus courante de carbone de la photosynthèse. Il est injecté dans le circuit gazeux, de telle sorte que le pH de la culture, sur lequel il influe et qui a tendance à augmenter sous l'effet de la photosynthèse, soit maintenu constant et égal à des valeurs comprises entre 6 et 8 selon les espèces cultivées. Pour cela, un capteur de pH 61 commande l'ouverture de l'électrovanne 62 ce qui a pour effet de laisser entrer du dioxyde de carbone dans le circuit gazeux de réacteur. Il passe au préalable dans un filtre coupant à 0,22 microns qui permet d'éviter les contaminations du milieu réactionnel.

Le milieu nutritif neuf est injecté dans le réacteur par une canalisation 63. Cette injection provoque l'élévation du niveau dans le réservoir **25** disposé au-dessus de l'élévateur à gaz **24**. Ce niveau doit être régulé faute de quoi la phase liquide serait aspirée par le compresseur **26**, ce qui pourrait causer divers dommages. Pour cela, un détecteur de niveau **64**, noté L, pilote l'ouverture de l'électrovanne de récolte **65**.

En outre, des moyens **66** sont prévus dans le distributeur **22** pour introduire des particules **15** dans le milieu réactionnel liquide, ou pour les retirer. Afin d'éviter que les particules **15** ne soient entraînées hors du réacteur, lors du soutirage, un tamis de maille légèrement inférieure à la taille des particules **15** est placé au raccordement **67**.

Un détecteur de turbidité à seuil **68** commande des pompes doseuses pour injecter le milieu nutritif neuf lorsque la culture atteint une certaine concentration. Ce détecteur de turbidité peut également agir sur la puissance des tubes incandescents 31, lorsque la concentration de microorganismes est inférieure à la concentration souhaitée.

Ces diverses régulations fonctionnent sans interférences entre elles et suffisent pour automatiser le réacteur pendant l'exploitation courante.

Par contre, la stérilisation initiale du photobioréacteur 18 et l'introduction de l'inoculum nécessitent des opérations manuelles difficiles à automatiser.

## Revendications

1. Procédé pour améliorer la vitesse de croissance de microorganismes phototrophes d'une culture en suspension dans un milieu réactionnel liquide, procédé selon lequel on soumet la culture à une réaction photosynthétique activée par des rayonnements traversant une paroi transparente (12, 32) d'une chambre (10) d'épaisseur d d'un photobioréacteur dans lequel circule le milieu réactionnel (11),
**caractérisé par le fait que** l'on règle l'absorbance de la culture en ajoutant au milieu réactionnel des particules (15) transparentes ou réfléchissantes ayant une densité égale à celle dudit milieu réactionnel, le pourcentage volumique desdites particules (15) dans le milieu réactionnel étant fonction de l'espèce de microorganismes cultivés, de l'épaisseur d de la chambre et de la concentration finale souhaitée de la culture dans le milieu réactionnel (11).

2. Procédé selon la revendication 1, dans lequel on fait circuler le milieu réactionnel (11) dans une boucle, on introduit un inoculum dans ladite boucle et on prélève une partie du milieu réactionnel, lorsque la concentration de culture atteint le seuil souhaité, **caractérisé par le fait que** l'on mesure la turbidité du milieu réactionnel et on règle l'intensité des rayonnements en fonction de la mesure de turbidité.

3. Procédé selon l'une quelconque des revendications 1 ou 2,
**caractérisé par le fait que** les particules (15) sont des billes pour éviter les effets de portance et de superposition.

4. Procédé selon l'une quelconque des revendications 1 à 3,
**caractérisé par le fait que** les particules (15) ont des tailles comprises entre 0,5mm et 10mm, en diamètre du cercle exinscrit.

5. Procédé selon l'une quelconque des revendications 1 à 4,
**caractérisé par le fait qu'**on utilise des particules (15) réalisées en enrobant des microbilles de verre creuses dans une matière plastique transparente dans des proportions telles que la densité du composite obtenu soit égale à celle du milieu réactionnel (11).

## Claims

1. A method of improving the rate of growth of phototropic microorganisms in a culture in suspension in a liquid reaction medium, in which method the culture is subjected to a photosynthesis reaction activated by radiation passing through a transparent wall (12, 32) of a chamber (10) of thickness d in a photobioreactor in which the reaction medium (11) circulates,
the method being **characterized by** the fact that the absorbance of the culture is adjusted by adding transparent or reflecting particles (15) to the reaction medium, which particles are of a density that is equal to that of said reaction medium, the volume percentage occupied by said particles (15) in the reaction medium being a function of the species of microorganism being cultured, of the thickness d of the chamber, and of the desired final concentration for the culture in the reaction medium (11).

2. A method according to claim 1, in which the reaction medium (11) is circulated round a loop, an inoculum is introduced into said loop, and a portion of the reaction medium is extracted once the concentration of the culture has reached the desired threshold, the method being **characterized by** the fact that the turbidity of the reaction medium is measured and the intensity of the radiation is adjusted as a function of the measured turbidity.

3. A method according to claim 1 or claim 2, **characterized by** the fact that the particles (15) are beads so as to avoid effects of lift and of superposition.

4. A method according to any one of claims 1 to 3, **characterized by** the fact that the particles (15) are of a size such that the diameter of the escribed circle lies in the range 0.5 mm to 10 mm.

5. A method according to any one of claims 1 to 4, **characterized by** the fact that the particles (15) used are made by coating hollow glass microbeads in a transparent plastics material at proportions such that the density of the resulting composite is equal to that of the reaction medium (11).

## Patentansprüche

1. Verfahren zur Verbesserung der Wachstumsgeschwindigkeit phototropher Mikroorganismen einer Suspensionskultur in einem flüssigen reaktiven Medium, wobei gemäß dem Verfahren die Kultur einer Photosynthesereaktion unterworfen wird, die durch Strahlung aktiviert wird, die eine transparente Wand (12, 32) einer Kammer (10) einer Dicke d eines Photobioreaktors durchdringt, in welchem das reaktive Medium (11) zirkuliert, **dadurch gekennzeichnet, daß** das Absorptionsvermögen der Kultur geregelt wird, indem dem reaktiven Medium transparente oder reflektierende Partikel (15) zugegeben werden, die eine gleiche Dichte wie das reaktive Medium aufweisen, wobei der Volumenanteil der Partikel (15) in dem reaktiven Medium von der Art der kultivierten Mikroorganismen, der Dicke d der Kammer und der gewünschten Endkonzentration der Kultur in dem reaktiven Medium (11) abhängig ist.

2. Verfahren nach Anspruch 1, bei welchem das reaktive Medium (11) in einem Kreis zirkuliert, ein Impfmaterial in den Kreis eingeführt wird und ein Teil des reaktiven Mediums entnommen wird, wenn die Konzentration der Kultur die gewünschte Schwelle erreicht, **dadurch gekennzeichnet, daß** die Trübung des reaktiven Mediums gemessen wird und die Intensität der Strahlung in Abhängigkeit von der Messung der Trübung geregelt wird.

3. Verfahren nach einem der Ansprüche 1 oder 2, **dadurch gekennzeichnet, daß** die Partikel (15) Kugeln sind, um die Effekte von Auftrieb und Überlagerung zu vermeiden.

4. Verfahren nach einem der Ansprüche 1 bis 3, **dadurch gekennzeichnet, daß** die Partikel (15) eine Größe zwischen 0,5 mm und 10 mm im Durchmesser des anliegend-tangierenden Kreises aufweisen.

5. Verfahren nach einem der Ansprüche 1 bis 4, **dadurch gekennzeichnet, daß** Partikel (15) verwendet werden, die hergestellt werden, indem hohle Mikrokugeln aus Glas mit einem transparenten Kunststoffmaterial in einem solchen Verhältnis überzogen werden, daß die Dichte des erhaltenen Komposits gleich derjenigen des reaktiven Mediums (11) ist.
